# EUROPEAN PATENT APPLICATION

(11) **EP 4 649 900 A1**
(43) Date of publication of application: **19.11.2025**
(21) Application number: 23915756.3
(22) Date of filing: 08.12.2023
(51) Int. Cl.: A61B 17/22, A61B 1/04, A61B 1/07

(54) **SHEATH PLACEMENT APPARATUS AND METHOD FOR USING SAME**

(30) Priority: 10.01.2023 CN 202310034222
(71) Applicant: Coastline Life Technologies (Suzhou), Co., Ltd., Suzhou, Jiangsu 215100 (CN)
(72) Inventor: SHI, Jun, Suzhou, Jiangsu 215100 (CN); ZHANG, Baoqing, Suzhou, Jiangsu 215100 (CN)
(74) Representative: Viering, Jentschura & Partner mbB Patent- und Rechtsanwälte
(86) International application number: PCT/CN2023/137367
(87) International publication number: WO 2024/148998

(57) **Abstract**

Disclosed are a sheath placement apparatus (100). In the present invention, the apparatus for sheath insertion (100) comprises: an inner layer member (1), wherein the inner layer member (1) is provided with an operation handle (11) and an insertion portion (12) connected to the operation handle (11), and the insertion portion (12) is provided with an imaging portion (123); an outer sheath (2), wherein a liquid drainage port (20) is formed on the outer sheath (2), the outer sheath (2) is provided with a distal open end (201), the insertion portion (12) is operably inserted into the outer sheath (2) and at least partially operably extends out of the distal open end (201), and the insertion portion (12) and the outer sheath (2) are operably spaced apart to form a first channel communicating the liquid drainage port (20) and the distal open end (201); and a middle layer member (3), wherein the middle layer member (3) is operably inserted between the outer sheath (2) and the insertion portion (12) and at least partially operably extends out of the distal open end (201), and when the middle layer member (3) exits from the space between the inner layer member (1) and the outer sheath (2), the first channel is formed between the inner layer member (1) and the outer sheath (2). The device reduces the costs of an operation while simplifying the steps of the operation, reduces the risk of the operation, and relieves the pain of patients.

## Description

This patent application claims the priority of the following Chinese patent applications:
Filed date: January 10, 2023; Application number: 2023100342224; Invention title: Apparatus for sheath Insertion and method of using the same;
the entire text of the above application is incorporated herein by reference.

### Technical field

The present invention relates to medical instruments, and in particular to an apparatus for sheath insertion and a method of using the same.

### Background

Existing treatments often require the use of medical equipment, such as lithotripsy treatment of ureteral stones, transoral cholangiopancreatoscopic lithotripsy treatment of gallstones, treatment of ureteral adhesions and cysts, etc., all of which require the equipment to be inserted into the lesion of the human body. However, existing equipment has various deficiencies.

For example, for the lithotripsy treatment of ureteral stones, two surgical methods, i.e. flexible ureteroscopic lithotripsy and rigid ureteroscopic lithotripsy, are currently used in clinical practice.

In rigid ureterolithotripsy, a rigid ureteroscope is used to reach the location of ureteral stones via the urethra and bladder, and a holmium laser is used for lithotripsy treatment. At present, the outer diameter of the conventional rigid ureteroscope is relatively large, during the operation, the ureter contracts due to the stimulation caused by the insertion of the rigid ureteroscope, and the space between the ureter and the rigid ureteroscope is very narrow, the liquid injected through the rigid ureteroscope during the operation is difficult to flow back. However, during holmium laser lithotripsy, the laser energy is converted into heat in addition to the lithotripsy, if there is no continuous water circulation, long-term lithotripsy can likely cause thermal damage to the ureter.

In order to avoid thermal damage to the ureter and form space for fluid to return during the operation, flexible ureteroscope lithotripsy is used. First, a rigid ureteroscope is used to inspect and initially dilate the affected ureter. And the rigid ureteroscope stops when it reaches a certain position in the ureter, then a guide wire is placed through the second channel of the rigid ureteroscope. After the rigid ureteroscope is withdrawn, the flexible ureteroscope sheath is placed along the guide wire. After the flexible ureteroscope sheath is successfully placed, a flexible ureteroscope is placed along the flexible ureteroscope sheath to complete lithotripsy treatment. However, as mentioned above, when using a flexible ureteroscopy to treat ureteral stones, a rigid ureteroscope and guide wire are required during the operation, and the surgical steps are cumbersome. At the same time, placing the ureteral guide sheath along the guidewire is an operation performed under indirect vision, which can likely cause artificial damage to the ureter, increase surgical risks and increase patient pain during the operation. And the cost is also high due to use of multiple equipment. In addition, during transoral cholangiopancreatoscopic lithotripsy treatment of gallstones, a duodenoscope is inserted through the mouth, a bile catheter is inserted into the opening of the pancreatic duct, and a contrast agent is injected and then X-rays are taken to visualize the bile and pancreatic ducts, and gallstones are removed through the mouth under the duodenoscope. If the diameter of the gallbladder stone is less than 1 cm, the endoscopic sphincterotomy (EST) can be used to remove the stone first. Gallstones with large diameters, confluence stones, and impacted stones are difficult to remove with mechanical lithotripsy, and a subscope is required. The subscope is pushed into the bile duct through the duodenal scope forceps channel and the laser is inserted into the working channel. Once reaching the stone site, laser lithotripsy can be performed under direct vision. Transoral cholangiopancreatoscopy technology does not require surgery, is less painful, highly reproducible, and the stones can be taken out multiple times.

However, transoral cholangiopancreatoscopic laser lithotripsy, as a relatively safe technology, still has adverse reactions or complications. During the operation, physiological saline is infused into the bile duct to maintain a clear field of vision, since there are no enough space water outlet due to the narrow bile duct and the thick endoscope, the pressure in the bile duct will increase, and the patient will experience nausea, vomiting, and postoperative diarrhea. If the perfusion amount of physiological saline is reduced during the operation, the stone powder during the lithotripsy will cause a decrease in visual field clarity, and it is very likely to damage the bile duct wall and bleed when the surgeon operate the laser, at the same time, the heat generated by continuous laser lithotripsy will cause thermal damage to the bile duct.

### Summary

The object of the present invention is to provide an apparatus for sheath insertion and a method of using the same, the system is easy to use, reduces surgical risks and relieves patient pain, and is low in cost.

In order to solve the above technical problems, embodiments of the present application provide an apparatus for sheath insertion, comprising:
an inner layer member, which has an operating handle and an insertion portion connected to the operating handle, and the insertion portion has an imaging portion;
an outer sheath, which is provided with a drainage port, and has a distal open end; the insertion portion is operably inserted into the outer sheath, and at least partially operably extends out of the distal open end; and the insertion portion and the outer sheath are operably spaced apart to form a first channel that communicates the drainage port and the distal open end; and
a middle layer member, which is operably inserted between the outer sheath and the insertion portion and at least partially operably extends out of the distal open end; when the middle layer member is withdrawn from the space between the inner layer member and the outer sheath, the first channel is formed between the inner layer member and the outer sheath.

Compared with the prior art, the embodiment of the present application assembles the inner layer member, the outer sheath and the middle layer member together, the camera function of the imaging portion can guide the system into the human body and reach the working area, and the middle layer member can be inserted between the outer sheath and the insertion portion to block the first channel between the inner layer member and the distal open end of the outer sheath, when the system enters into the human body, no foreign matter will enter into the first channel through the distal open end, nor will the tissue be bruised by the presence of the distal open end. When the system reaches the working position, the middle layer member can be evacuated from the outer sheath, so as to make the first channel unobstructed, and working instruments can pass through the first channel to perform work such as lithotripsy and drainage, and liquid can enter into the channel from the distal open end and be drained from the drainage port, allowing the system to be used smoothly. It can be seen from the above that this system has fewer components and a simple structure, and does not require multiple components to enter and exit the human body multiple times in steps and has the first channel for liquid to pass through, it is simple and convenient to operate, saves operation time, is visible throughout the operation, and is safe to operate, making the surgery safer, less painful for the patient, and less expensive.

In an embodiment, the insertion portion is provided with a second channel extending along an extension direction of the insertion portion, and the second channel runs through the insertion portion.

In an embodiment, the middle layer member is detachably connected to the inner layer member.

In an embodiment, the middle layer member is a middle layer dilation tube, the insertion portion is operably inserted into or extracted from the middle layer dilation tube; and the middle layer dilation tube is detachably connected to the insertion portion.

In an embodiment, a portion of the middle layer member located between the inner layer member and the outer sheath operably fills an area between the inner layer member and the distal open end of the outer sheath.

In an embodiment, the middle layer dilation tube has a tube body and a connecting adapter connected to the tube body;
the insertion portion has a docking portion connected to the connecting adapter, and the docking portion is close to the operating handle; when the insertion portion, the outer sheath and the middle layer dilation tube are assembled together, the connecting adapter is located between the outer sheath and the operating handle.

In an embodiment, the insertion portion has a encapsulating sheath, and an end of the encapsulating sheath away from the operating handle has a first rounded guide surface, and the first rounded guide surface gradually converges toward an axis of the insertion portion in a direction away from the operating handle.

In an embodiment, an end of the middle layer dilation tube away from the operating handle has a second rounded guide surface, and the second rounded guide surface gradually converges toward an axis of the middle layer dilation tube in a direction away from the operating handle.

In an embodiment, the first rounded guide surface and the second rounded guide surface are adjacent to each other and extend into a same plane.

In an embodiment, the outer sheath has a first sheath body and a second sheath body extending from the first sheath body, the drainage port is provided on the first sheath body, and the distal open end is provided on the second sheath body; wherein the diameter of the cross section of the second sheath body gradually decreases in a direction away from the first sheath body.

In an embodiment, a drainage tube communicating with the first channel protrudingly extends from the side wall of the outer sheath, and the drainage port is provided on the drainage tube.

In an embodiment, the insertion portion has a encapsulating sheath, and a light source portion and the imaging portion provided in the encapsulating sheath; the second channel runs through the encapsulating sheath;
an end of the encapsulating sheath away from the operating handle is partially opened and communicates with an inner cavity of the encapsulating sheath, and an end of the light source portion and the imaging portion are located at an end of the sheath away from the operating handle;
wherein, the operating handle is provided with a liquid inlet extension tube, which is communicated with an inner cavity of the encapsulating sheath.

In an embodiment, when the middle layer member is located between the outer sheath and the inner layer member and reaches the working position, the middle layer member is tightly connected to the outer sheath and the inner layer member.

In an embodiment, the outer sheath has a hydrophilic coating, and the outer sheath is made of polymer material; the middle layer member is made of polymer material or metal material, and the middle layer member is rigid.

In an embodiment, the outer sheath has a proximal open end, and the insertion portion is at least partially located outside the proximal open end.

In an embodiment, the apparatus for sheath insertion is a natural orifice apparatus for sheath insertion.

An embodiment of the present application provides a natural orifice apparatus for sheath insertion, comprising an apparatus for sheath insertion as described in any one of the above.

An embodiment of the present application provides a method of using an apparatus for sheath insertion, which uses a apparatus for sheath insertion described in any one of the above, comprising the following steps:
inserting the inner layer member into the middle layer member;
inserting the middle layer member inserted with the inner layer member into the outer sheath;
inserting the outer sheath, the middle layer member and the inner layer member assembled together into the working area;
withdrawing the middle layer member and the inner layer member together from the outer sheath;
separating the inner layer member from the middle layer member;
inserting the inner layer member into the outer sheath.

In an embodiment, in the step of inserting the middle layer member inserted with the inner layer member into the outer sheath, the middle layer member extends from the distal open end.

In an embodiment, during the step of inserting the outer sheath, the middle layer member and the inner layer member assembled together into the working area, the imaging portion operates throughout the process.

### Brief description of drawings

One or more embodiments are exemplified by the pictures in the corresponding drawings, which do not constitute limitations to the embodiments. Elements with the same reference numerals in the drawings are represented as similar elements, unless otherwise stated, the figures in the drawings are not intended to be limited to scale.
FIG.1 is a schematic structural diagram of an apparatus for sheath insertion according to an embodiment of the present invention;
FIG.2 is a partial enlarged view of A in FIG.1;
FIG.3 is an exploded view of an apparatus for sheath insertion according to an embodiment of the present invention;
FIG.4 is a cross-sectional view of an apparatus for sheath insertion according to an embodiment of the present invention;
FIG.5 is a partial enlarged view of B in FIG.4;
FIG.6 is a partial enlarged view of C in FIG.4;
FIG.7 is an enlarged view of the end of the apparatus for sheath insertion inserted into the human body according to one embodiment of the present invention;
FIG.8 is a method of using the apparatus for sheath insertion according to an embodiment of the present invention;
   in which, 100. apparatus for sheath insertion; 1. inner layer member; 11. operating handle; 12. insertion portion; 121. encapsulating sheath; 122. light source portion; 123. imaging portion; 124. docking portion; 2. outer sheath; 21. first sheath body; 22. second sheath body; 20. drainage port; 201. distal open end; 202. proximal open end; 3. middle layer dilation tube; 31. tube body; 32. connecting adapter; 4. first rounded guide surface; 5. second rounded guide surface; 6. drainage tube; 7. cable; 8. second channel; 91. first interface; 92. second interface.

### Detailed description of embodiments

In order to make the objectives, technical solutions and advantages of the embodiments of the present invention clearer, each embodiment of the present invention will be described in detail below with reference to the accompanying drawings. However, those of ordinary skill in the art will understand that in various embodiments of the present invention, many technical details are provided to enable readers to better understand the present application. However, even without these technical details and various changes and modifications based on the following embodiments, the technical solution claimed in this application can also be implemented.

In the following description, for the purpose of explaining the various disclosed embodiments, certain specific details are set forth in order to provide a thorough understanding of the various disclosed embodiments. However, one skilled in the relevant art will recognize that the embodiments may be practiced without one or more of these specific details. In other cases, The well-known devices, structures, and technologies associated with this application may not be shown or described in detail to avoid unnecessary confusion with the description of the embodiments.

Unless the context requires otherwise, throughout the specification and claims, the word "include" and variations thereof, such as "comprises" and "has" are to be understood in an open, inclusive sense, that is, to mean "including, but not limited to". Various embodiments of the present invention will be described in detail below with reference to the accompanying drawings, so that the purpose, features and advantages of the present invention can be more clearly understood. It should be understood that the embodiments shown in the drawings do not limit the scope of the present invention, but are only used to illustrate the essential spirit of the technical solution of the present invention.

Reference throughout this specification to "one embodiment" or "an embodiment" means that a particular feature, structure, or characteristic described in connection with the embodiment is included in at least one embodiment. Thus, the appearances of "in one embodiment" or "an embodiment" in various places throughout this specification are not necessarily all referring to the same embodiment. Additionally, specific features, structures, or characteristics may be combined in any manner in one or more embodiments.

As used in this specification and the appended claims, the singular forms "a" and "the" include plural referents, unless the context clearly dictates otherwise. It should be noted that the term "or" is generally used in its sense including "and/or", unless the context clearly dictates otherwise.

In the following description, in order to clearly demonstrate the structure and working mode of the present invention, many directional words will be used to describe it, but "front", "back", "left", "right", "outside", "inside", "outward", "inward", "up", "down" and other words should be understood as convenient terms and should not be understood as limiting terms.

Embodiments of the present invention are described below with reference to the accompanying drawings.

An embodiment of the present invention relates to an apparatus for sheath insertion. The apparatus for sheath insertion can be a natural orifice apparatus for sheath insertion, for example, used in the ureter, the following takes the apparatus for sheath insertion used in the ureter as an example for illustration. It can be understood that the apparatus for sheath insertion may also be used in other cases, such as in renal cysts or gallstone lithotripsy.

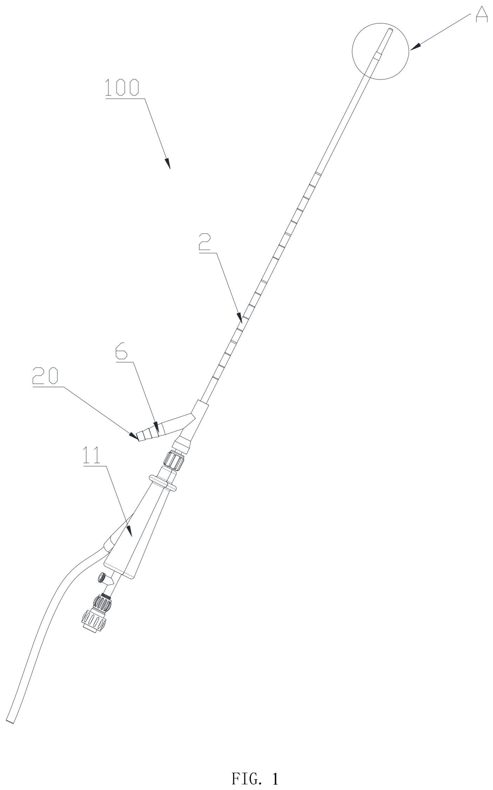

As shown in FIGS.1, 3 and 4, the apparatus for sheath insertion 100 includes: an inner layer member 1, an outer sheath 2 and a middle layer dilation tube 3. The inner layer member 1 has an operating handle 11 and an insertion portion 12 connected to the operating handle 11. The insertion portion 12 is provided with a second channel 8 extending along the extension direction of the insertion portion 12 therein. The second channel 8 runs through the insertion portion 12, and working instruments such as laser fibers, occluders or basket can be inserted into the second channel. Of course, it can be used without any other instruments inserted. at this time, the insertion portion 12 may be a shell of polymer material or a shell of other materials. The outer sheath 2 is provided with a drainage port 20, and an end of the outer sheath 2 is a distal open end 201. The insertion portion 12 is inserted into the outer sheath 2, and the insertion portion 12 at least partially operably extends out of the distal open end 201. The operating handle 11 is located outside the insertion portion 12. The diameter of the insertion portion 12 is smaller than the inner diameter of the outer sheath 2, and the insertion portion 12 and the outer sheath 2 can be spaced apart to form a first channel communicating with the drainage port 20 and the distal open end 201. The insertion portion 12 can be inserted into the middle layer dilation tube 3. The insertion portion 12 and the middle layer dilation tube 3 are inserted into the outer sheath 2 together. When the middle layer dilation tube 3 is inserted into the outer sheath 2, it blocks the first channel between the inner layer member 1 and the distal open end 201 of the outer sheath 2, and the middle dilation tube 3 can partially extend out of the distal open end 201 together with the inner layer member 1. It can be understood that in some embodiments, there may be a partial gap between the middle layer member and the outer sheath 2 or the inner layer member 1. In addition, in some embodiments, there may be no second channel in the insertion portion, and no instrument can be inserted, for example, the insertion portion 12 itself is an optical fiber, and the imaging portion may be a camera provided at the end of the optical fiber.

Preferably, after the middle layer dilation tube 3 is inserted into the outer sheath 2 and reaches the working position, the middle layer dilation tube 3 is tightly connected to the outer sheath or the inner layer member 1, that is, the middle layer dilation tube 3 and the inner layer member 1 are in close contact with each other, the middle layer dilation tube 3 is in close contact with the outer sheath 2 or the middle layer dilation tube 3 is in close contact with the distal open end of the outer sheath 2. The working position is the position where the middle layer member can be inserted into the human body for work after assembled with the outer sheath 2 and the inner layer member 1 together.

Specifically, as shown in FIGS.1, 2, 3 and 4, when inserting the apparatus for sheath insertion 100 into the ureter, the middle layer dilation tube 3 is first sleeved over the inner layer member 1, and the middle layer dilation tube 3 and the inner layer member 1 are inserted into the outer sheath 2 together, and the inner layer member 1, the outer sheath 2 and the middle layer dilation tube 3 enter into the ureter together. When entering into the ureter, the inner layer member 1 is used for camera guidance to find the location of the stone, which is the working area. After reaching the working position, the middle layer dilation tube 3 is sleeved over the inner layer member 1 and extracted from the outer sheath 2 together, and then the middle layer dilation tube 3 is withdrawn from the inner layer member 1, and the inner layer member 1 is inserted into the outer sheath 2 alone, at this time, there is a gap between the inner layer member 1 and the outer sheath 2 to form a first channel. The inner layer member 1 extends out of the distal open end 201, and the working instrument extends out of the second channel 8 to perform lithotripsy. During lithotripsy, water or other liquid flows from the inner layer member 1 into the working area, then flows into the first channel from the distal open end 201, and flows from the first channel into the drainage port 20 for discharge. Of course, the inner layer member has a certain degree of toughness, and the portion of the inner layer member extending from the distal open end 201 can be bent and turned. It can be understood that the apparatus for sheath insertion 100 can break stones in other areas besides entering into the ureter for lithotripsy, and is not limited to the lithotripsy in the ureter involved in this embodiment. Alternatively, when the liquid is discharged from the human body, it is also feasible to flow out from the second channel 8, at this time, the space between the outer sheath 2 and the inner layer member 1 is used as liquid inlet.

From the above, it can be seen that the inner layer member 1, the outer sheath 2 and the middle layer member 3 are assembled together, the camera function of the inner layer member 1 can guide the system into the ureter and reach the working area, and the middle layer member 3 can be inserted between the outer sheath 2 and the insertion portions 12 to block the first channel between the inner layer member 1 and the distal open end 201 of the outer sheath 2, when the system enters into the ureter, no foreign matter will enter the first channel through the distal open end 201, and the tissue will not be bruised due to the presence of the distal open end 201. When the system reaches the working position, the middle layer member can be evacuated from the outer sheath 2, so as to make the first channel unobstructed, and working instruments can pass through the second channel 8 to perform work such as lithotripsy. And during lithotripsy, and liquid can enter into the first channel from the distal open end 201 and be drained from the drainage port 20, allowing the system to be used smoothly. It can be seen from the above that this system has fewer components and a simple structure, and does not require multiple components to enter and exit the ureter multiple times in steps. It is simple and convenient to operate, saves operation time, is visible throughout the operation, and is safe to operate, making the surgery safer, less painful for the patient, and less expensive.

It can be understood that in this embodiment, the middle layer dilation tube 3 is a hollow tube, into which the inner layer member 1 can be inserted. In other embodiments, the middle layer dilation tube 3 can be a rod-shaped or other structural member that may be inserted into the outer sheath 2 side by side with the inner layer member 1. At this time, the middle layer dilation tube 3 can be called as the middle layer member, when the apparatus for sheath insertion 100 enters into the ureter, the middle layer member can block the first channel between the inner layer member 1 and the outer sheath 2 at the distal open end 201.

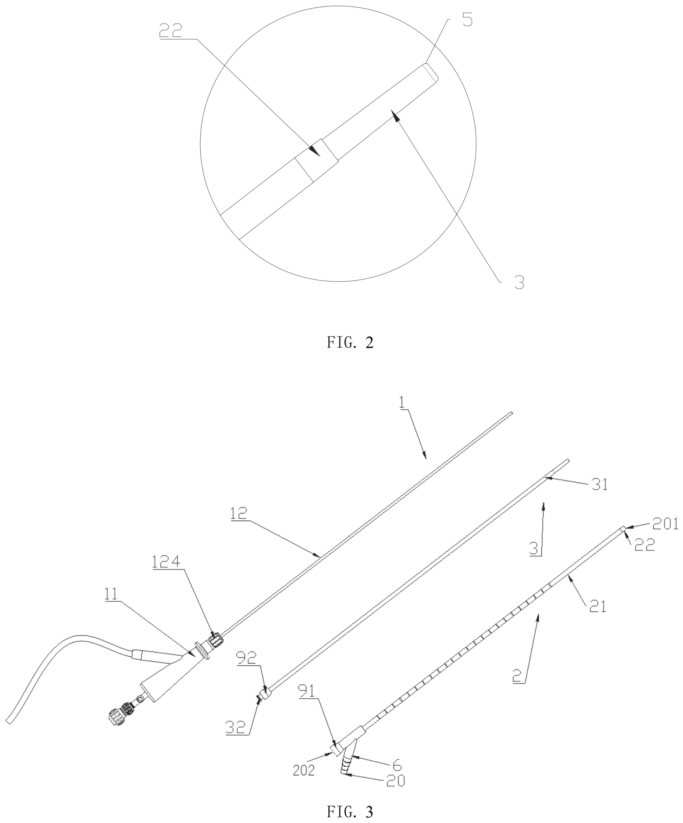

Further, as shown in FIGS. 1 and 3, the middle layer dilation tube 3 is detachably connected to the inner layer member 1. Specifically, when the apparatus for sheath insertion 100 is inserted into the ureter, the middle layer dilation tube 3 and the inner layer member 1 are fixed together to prevent the middle layer dilation tube 3 from being displaced during the operation, making the operation safer and more reliable. It can be understood that in other embodiments, the middle layer dilation tube 3 can also be detachably connected to the outer sheath 2.

Further, the insertion portion 12 is operably inserted into or extracted from the middle layer dilation tube 3, and the middle layer dilation tube 3 is detachably connected to the insertion portion 12. It can be understood that in other embodiments, the middle layer dilation tube 3 can also be detachably connected to the operating handle 11 . Specifically, as shown in FIGS. 1, 3 and 4, the middle layer dilation tube 3 has a tube body 31 and a connecting adapter 32 connected to the tube body 31. The insertion portion 12 has a docking portion 124 connected to the connecting adapter 32, and the docking portion 124 is close to the operating handle 11. When the insertion portion 12 , the outer sheath 2 , and the middle layer dilation tube 3 are assembled together, the connecting adapter 32 is located between the outer sheath 2 and the operating handle 11. In order to position the middle layer dilation tube 3 and the outer sheath 2 relatively, the outer sheath 2 has a first interface 91 at one end close to the operating handle 11, and the first interface 91 is mated with a second interface 92 on the middle layer dilation tube 3, and the second interface 92 is embedded in the first interface 91 and matches the inner contour of the first interface 91. The first interface 91 and the second interface 92 both have portions with inner diameters that gradually increase in a direction toward the operating handle 11. The docking portion 124 may be threadedly connected to the insertion portion 12, after the insertion portion 12, the outer sheath 2 and the middle layer dilation tube 3 are inserted into the working position, the insertion portion 12 and the middle layer dilation tube 3 are extracted from the outer sheath 2 together, and then the middle layer dilation tube 3 is unscrewed from the insertion portion 12, and the middle layer dilation tube 3 is extracted from the end of the insertion portion 12 away from the operating handle 11.

It can be understood that in other embodiments, the docking portion 124 and the insertion portion 12 are connected through a snap connector, for example, there are protrusions on the docking portion 124 and notches on the insertion portion 12, and the protrusions can be embedded into the notches. In one embodiment, the insertion portion 12 and the connecting adapter 32 are connected through a Luer interface. That is to say, the middle layer member and the inner layer member can be fixedly connected.

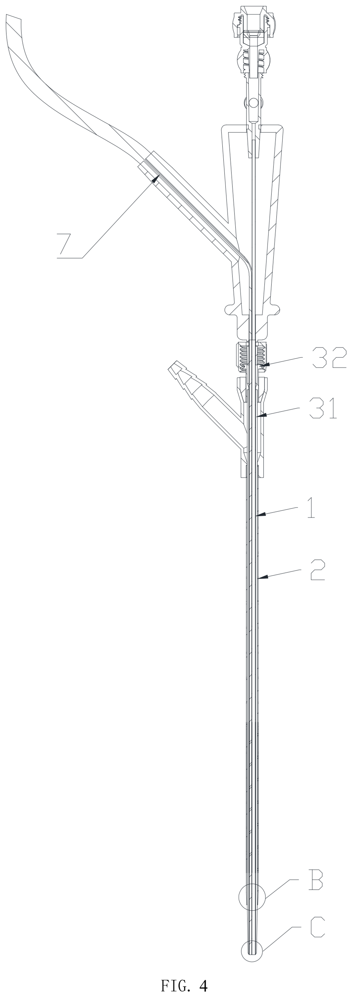

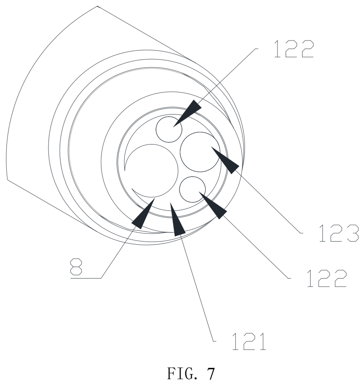

In addition, as shown in FIGS. 4 and 7, the insertion portion 12 has a encapsulating sheath 121, and a light source portion 122 and an imaging portion 123 provided in the encapsulating sheath 121. The end of the encapsulating sheath 121 away from the operating handle 11 is partially open and communicates with the inner cavity of the encapsulating sheath 121, and one end of the light source portion 122 and the imaging portion 123 are located at the end of the encapsulating sheath 121 away from the operating handle 11. The second channel 8 runs through the encapsulating sheath 121. The operating handle 11 is provided with a liquid inlet extension tube, and the liquid inlet extension tube communicates with the inner cavity of the encapsulating sheath 121. The light source portion 122 can be a light guide fiber, and the imaging portion 123 includes a lens, the light guide fiber and one end of the lens are located at the end of the encapsulating sheath 121 away from the operating handle 11, light is provided for the light source portion 122 through an illuminating member, a conduit fiber 122 may be extended in the cable 7, and there are also signal transmission wires in the cable that supply power to the imaging portion 123 and the illuminating member. Specifically, the light source portion 122 illuminates through the end of the the encapsulating sheath 121 entering into the human body, and working instruments such as laser fibers or guide wires can be protruded from the second channel 8 for work. Since the light source portion 122 is provided, the light source portion 122 has relatively good toughness and low cost, and the light source portion 122 is not easily broken. The encapsulating sheath 121 of the inner layer member 1 can be a soft sheath. The arrangement of the light source portion 122 makes the whole quality of the inner layer member 1 guaranteed during use and not easily damaged. In addition, in some embodiments, the light source portion 122 includes an LED light, which is directly provided at the end of the encapsulating sheath 121 away from the operating handle 11; or in some embodiments, there may be no second channel inside the insertion portion, that is, there is no encapsulating sheath 121 in the insertion portion, no instrument can be inserted. For example, the insertion portion 12 itself is an optical fiber, and the imaging portion may be a camera installed at the end of the optical fiber. As shown in FIGS. 2, 4 and 6, in order to reduce damage to the human body when the insertion portion 12 is inserted into the human body, the end of the sheath 121 away from the operating handle 11 has a first rounded guide surface 4, and the first rounded guide surface gradually converges toward the axis of the insertion portion 12 in a direction away from the operating handle 11. The middle layer dilation tube 3 and the insertion portion 12 partially extend out of the distal open end 201 of the outer sheath 2, and the end of the middle layer dilation tube 3 will directly contact the human body, in order to reduce damage to the human body, the end of the middle layer dilation tube 3 away from the operating handle 11 has a second rounded guide surface 5, and the second rounded guide surface 5 gradually converges toward the axis of the middle layer dilation tube 3 in a direction away from the operating handle 11.

Preferably, the first rounded guide surface and the second rounded guide surface 5 are adjacent to each other and extend into the same plane. That is to say, when the middle layer dilation tube 3 and the insertion portion 12 are sleeved together and inserted into the human body, portion of the insertion portion 12 extends out of the end of the middle layer dilation tube 3 extending out of the outer sheath 2, and the extension line of the second rounded guide surface 5 extends on the first rounded guide surface, causing the portion of the insertion portion 12 extending outside the middle layer dilation tube 3 to form a smooth transition with the second rounded guide surface 5, which makes the entry into the human body smoother and does not cause any damage to human tissue.

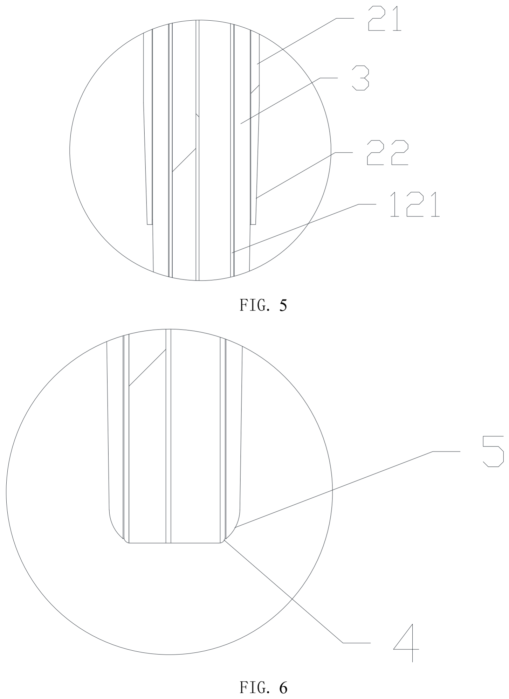

Further, as shown in FIGS. 4 and 5 , the outer sheath 2 has a first sheath body 21 and a second sheath body 22 extending from the first sheath body 21, the drainage port 20 is provided on the first sheath body 21, and the open end 201 is provided on the second sheath body 22, wherein the diameter of the cross-section of the second sheath body 22 gradually decreases in a direction away from the first sheath body 21, that is, the outer diameter of the second sheath body 22 gradually decreases toward the distal open end 201, and the inner diameter remains unchanged. Preferably, the outer diameter of the second sheath body 22 gradually decreases toward the distal open end 201, so that the outer surface of the second sheath body 22, the first rounded guide surface and the second rounded guide surface 5 are adjacent to each other and extend into the same plane.

In addition, a drainage tube 6 communicating with the first channel protrudes and extends from the side wall of the outer sheath 2 , and the drainage port 20 is provided on the drainage tube 6.

Furthermore, the inner diameter of the outer sheath 2 along its extension direction is same, and the outer diameter of the portion of the insertion portion 12 located in the outer sheath 2 along its extension direction is same, when the insertion portion 12 is located within the outer sheath 2, the first channel between the insertion portion 12 and the outer sheath 2 extends along the length direction of the outer sheath 2, and the space of the first channel along its extending direction remains uniform, allowing the liquid flowing out of the inner layer member 1 to flow out smoothly through the first channel.

In this embodiment, the illumination in the inner layer member 1 uses the optical fiber, the encapsulating sheath 121 is a soft sheath, and the inner layer member 1 can be a disposable material. The middle layer dilation tube 3 is rigid and can be made of polymer material or metal material, and is also a disposable material. Similarly, the outer sheath 2 is also a disposable material.

Further, the outer sheath 2 has a proximal open end 202, and the insertion portion is at least partially located outside the proximal open end 202.

Further, the outer sheath has a hydrophilic coating, and the outer sheath is made of polymer material.

It can be understood that in other embodiments, the apparatus for sheath insertion can be used for gallstone lithotripsy or renal cyst. Before gallstone lithotripsy, the middle layer dilation tube 3 is withdrawn. Since the channel between the inner layer member 1 and the outer sheath 2 can be used in conjunction with the second channel 8 to inject physiological saline or water and then discharge the used water, thereby reducing the risk of surgery and relieving the patient's pain. Alternatively, the apparatus for sheath insertion is used in renal cysts, the medicine is injected through the second channel 8 of the inner layer member 1, and the syringe draws out the cyst fluid through the drainage port and the cyst fluid flows out from the space between the inner layer member 1 and the outer sheath 2.

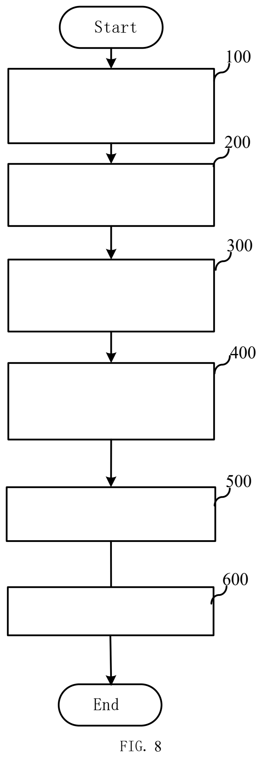

Another embodiment of the present application also provides a method of using an apparatus for sheath insertion. As shown in FIGs. 3 and 8, using the above apparatus for sheath insertion includes the following steps:
Step 100, inserting the inner layer member into the middle layer member 1;
Step 200, inserting the middle layer member inserted with the inner layer member 1 into the outer sheath 2;
Step 300, inserting the outer sheath 2, middle layer member and inner layer member 1 assembled together into the working area;
Step 400, withdrawing the middle layer member and the inner layer member 1 together from the outer sheath 2;
Step 500, separating the middle layer member from the inner layer member 1, that is, extracting the inner layer member 1 from the middle layer member.
Step 600, inserting the inner layer member into the outer sheath 2.

Specifically, the cooperation of the middle layer member with the inner layer member 1 and the outer sheath 2 is mentioned in the above embodiment of the apparatus for sheath insertion, and will not be described in detail here.

Further, in step 200, the mid-layer member extends out of the distal open end 201. Further, during the process of step 300, the imaging portion 123 operates throughout the process.

The preferred embodiments of the present invention have been described in detail above, but it should be understood that aspects of the embodiments can be modified if necessary to employ aspects, features and concepts from various patents, applications and publications to provide additional embodiments.

These and other changes can be made to the embodiments in view of the above detailed description. In general, in the claims, the terms used should not be construed as limiting to the specific embodiments disclosed in the specification and claims, but should be understood to include all possible embodiments together with all equivalent scope enjoyed by these claims.

Those of ordinary skill in the art can understand that the above-mentioned embodiments are specific examples for implementing the present invention, however in practical applications, various changes can be made in form and details without departing from the spirit and scope of the invention.

## Claims

1. An apparatus for sheath insertion, **characterized in that** comprising:
an inner layer member, which has an operating handle and an insertion portion connected to the operating handle, and the insertion portion has an imaging portion;
an outer sheath, which is provided with a drainage port, and has a distal open end; the insertion portion is operably inserted into the outer sheath, and at least partially operably extends out of the distal open end; and the insertion portion and the outer sheath are operably spaced apart to form a first channel that communicates the drainage port and the distal open end; and
a middle layer member, which is operably inserted between the outer sheath and the insertion portion and at least partially operably extends out of the distal open end; when the middle layer member is withdrawn from the space between the inner layer member and the outer sheath, the first channel is formed between the inner layer member and the outer sheath.

2. The apparatus for sheath insertion according to claim 1, wherein the inserting portion is provided with a second passage extending along an extending direction of the inserting portion, and the second passage runs through the inserting portion.

3. The apparatus for sheath insertion according to claim 1, wherein the middle layer member is detachably connected to the inner layer member.

4. The apparatus for sheath insertion according to claim 2, wherein the middle layer member is a middle layer dilation tube, and the insertion portion can be operably inserted into or extracted from the middle layer dilation tube; and the middle layer dilation tube is detachably connected to the insertion portion.

5. The apparatus for sheath insertion according to claim 4, wherein a portion of the middle layer member between the inner layer member and the outer sheath operably fills an area between the inner layer and the distal open end of the outer sheath.

6. The apparatus for sheath insertion according to claim 4, wherein the middle layer dilation tube has a tube body and a connecting adapter connected to the tube body; the insertion portion has a docking portion connected to the connecting adapter, and the docking portion is close to the operating handle.

7. The apparatus for sheath insertion according to claim 4, wherein the insertion portion has a encapsulating sheath, and an end of the encapsulating sheath away from the operating handle has a first rounded guide surface, and the first rounded guide surface gradually converges toward an axis of the insertion portion in a direction away from the operating handle.

8. The apparatus for sheath insertion according to claim 7, wherein an end of the middle layer dilation tube away from the operating handle has a second rounded guide surface, and the second rounded guide surface gradually converges toward an axis of the middle layer dilation tube in a direction away from the operating handle.

9. The apparatus for sheath insertion according to claim 8, wherein the first rounded guide surface and the second rounded guide surface are adjacent to each other and extend into the same plane.

10. The apparatus for sheath insertion according to claim 1, wherein the outer sheath has a first sheath body and a second sheath body extending from the first sheath body, the drainage port is provided on the first sheath body, and the distal open end is provided on the second sheath body; wherein the diameter of the cross section of the second sheath body gradually decreases in a direction away from the first sheath body.

11. The apparatus for sheath insertion according to claim 1, wherein a drainage tube communicating with the first channel protrudingly extends from the side wall of the outer sheath, and the drainage port is provided on the drainage tube.

12. The apparatus for sheath insertion according to claim 2, wherein the insertion portion has a encapsulating sheath, and a light source portion and the imaging portion provided in the encapsulating sheath; the second channel runs through the encapsulating sheath;
an end of the encapsulating sheath away from the operating handle is partially opened and communicates with an inner cavity of the encapsulating sheath, and an end of the light source portion and the imaging portion are located at the end of the encapsulating sheath away from the operating handle;
wherein the operating handle is provided with a liquid inlet extension tube, which is communicated with the inner cavity of the encapsulating sheath.

13. The apparatus for sheath insertion according to claim 1, wherein when the middle layer member is located between the outer sheath and the inner layer member and reaches the working position, the middle layer member is tightly connected to the outer sheath and the inner layer member.

14. The apparatus for sheath insertion according to claim 1, wherein the outer sheath has a hydrophilic coating and is made of a polymer material; the middle layer member is made of a polymer material or a metal material, and the middle layer member is rigid.

15. The apparatus for sheath insertion according to claim 1, wherein the outer sheath has a proximal open end, and the insertion portion is at least partially located outside the proximal open end.

16. The apparatus for sheath insertion according to claim 1, wherein the apparatus for sheath insertion is a natural orifice apparatus for sheath insertion.

17. A method of using an apparatus for sheath insertion, **characterized in that** using the apparatus for sheath insertion according to any one of claims 1 to 16, and comprising the following steps:
inserting the inner layer member into the middle layer member;
inserting the middle layer member inserted with the inner layer member into the outer sheath;
inserting the outer sheath, the middle layer member and the inner layer member assembled together into a working area;
withdrawing the middle layer member and the inner layer member together from the outer sheath;
separating the inner layer member from the middle layer member;
inserting the inner layer member into the outer sheath.

18. The method for using the apparatus for sheath insertion according to claim 17, wherein
in the step of inserting the middle layer member inserted with the inner layer member into the outer sheath, the middle layer member extends out of the distal open end.

19. The method for using the apparatus for sheath insertion according to claim 17, wherein
during the step of inserting the outer sheath, the middle layer member and the inner layer member assembled together into the working area, the imaging portion operates throughout the process.
